# EUROPEAN PATENT APPLICATION

(11) **EP 2 468 192 A1**
(43) Date of publication of application: **27.06.2012**
(21) Application number: 11193738.9
(22) Date of filing: 15.12.2011
(51) Int. Cl.: A61B 17/02, A61B 17/34

(54) **Surgical retractor having ring of variable durometer**

(30) Priority: 21.12.2010 US 201061425400 P; 09.09.2011 US 201113228960
(71) Applicant: Tyco Healthcare Group LP, Mansfield, MA 02048 (US)
(72) Inventor: Pribanic, Russell, Roxbury, Connecticut 06783 (US)
(74) Representative: Soames, Candida Jane

(57) **Abstract**

A surgical retractor includes a tubular member having a proximal end and a distal end, a first ring secured at the proximal end of the tubular member and a second ring secured at the distal end of the tubular member. The first ring is formed by combining a first material having a first durometer and a second material having a second durometer. Alternatively, a flexible sealing ring for use with a surgical retractor to prevent cross-contamination during surgery of fluids and solids is presented, the ring composed of at least two materials, the first material having a first durometer and the second material having a second durometer, where the first durometer is greater than the second durometer.

## Description

### CROSS REFERENCE TO RELATED APPLICATION

The present application claims the benefit of and priority to U. S. Provisional Application Serial No. 61/425,400 filed on December 21, 2010, the entire contents of which are incorporated herein by reference.

### BACKGROUND

### Technical Field

The present disclosure relates to a surgical retractor, and more particularly, to a surgical retractor having at least one ring of variable durometer.

### Background of Related Art

Adequate anatomical exposure is required to allow surgical procedures to be safely and effectively performed. Anatomical exposure is achieved by separating the walls of a natural orifice or spreading apart the margins of a surgical incision. A difficult surgical procedure may be simplified by adequate retraction, whereas a relatively simple surgical procedure may be made more difficult or even dangerous by the lack of adequate retraction. Exposure is maximized with correct incision placement and well directed retraction.

Retraction may be achieved in several different ways. The most common method of surgical wound retraction is by the use of hand held retractors. These may be made of metal or thermoplastics and allow an operator to apply a retraction force to the wound edges. They are disposable or reusable and come in a variety of shapes and sizes to satisfy the requirements of different surgical procedures.

Another type of retractor is a frame mounted retractor device. Such devices consist of a rigid circular or horseshoe-shaped frame on which multiple, detachable and movable paddle retractors are attached. The device may be mounted to an operating table to provide secure anchorage. Retraction may be applied in required directions. Typically such retractors are made of stainless steel to facilitate cleaning and sterilization for reuse.

Additionally, various surgical procedures are performed in a minimally invasive manner. This includes forming a small incision through a body wall of the patient and inserting an access port through the incision to protect the wound created by the incision and provide a pathway for the insertion of surgical instruments. The access port generally includes a flexible tubular member with opposed first and second rings.

When choosing an access port, care must be taken to ensure that the access port has sufficient length to completely pass through the body wall of the patient. Additionally, the access port must be chosen such that it does not extend too far into the body cavity and obstruct the surgical procedure. Often times it is necessary to access a large area of the body cavity with a minimal number of access ports. In this situation, the surgeon is often limited by the angle at which the surgeon may insert surgical instruments through the access port. Further, care must be taken to prevent movement of the access port during the surgical procedure.

It is thus desirable to provide a surgical access port or surgical retractor, which may be firmly stabilized or secured about the penetrated tissue.

### SUMMARY

A surgical retractor is disclosed herein. The surgical retractor includes a tubular member having a proximal end and a distal end, a first ring secured at the proximal end of the tubular member and a second ring secured at the distal end of the tubular member. The first ring is formed by combining a first material having a first durometer and a second material having a second durometer.

Additionally, the first ring and the second ring are O-rings and are flexible. The tubular member is a sleeve or a liner, and may be flexible. The proximal ring is formed by combining a first material having a first durometer and a second material having a second durometer. The second ring is formed by combining the first material having the first durometer and the second material having the second durometer.

The first material defines an outer periphery of the first ring and the second material defines an inner periphery of the first ring. The first durometer may be greater than the second durometer or vice versa. The first material may have a hardness in a range of about 70-100 on a Shore A durometer, whereas the second material may have a hardness in a range of about 40-70 on a Shore A durometer.

Moreover, in example embodiments, the second material defines an inner tubular portion of the first ring and the first material defines an outer tubular portion of the first ring by encircling at least a portion of the second material. In other example embodiments, the first material combines with the second material so that a hollow inner portion is defined within the first ring.

A wound protector is also disclosed herein. The wound protector includes a distal ring, a proximal ring and a flexible sleeve extending between the proximal ring and the distal ring.

A flexible sealing ring for use with a surgical retractor to prevent cross-contamination during surgery of fluids and solids is also presented, the ring composed of at least two materials, the first material having a first durometer and the second material having a second durometer, where the first durometer is greater than the second durometer.

### DESCRIPTION OF THE DRAWINGS

The accompanying drawings, which are incorporated in and form part of the specification, illustrate the present disclosure when viewed with reference to the description, wherein:

FIG. 1 is a perspective view of a surgical retractor illustrating at least one ring, in accordance with the present disclosure;

FIG. 2A is a perspective view of the surgical retractor of FIG. 1 partially inserted through tissue, in accordance with the present disclosure;

FIG. 2B is a perspective view of the surgical retractor of FIG. 1 fully inserted through tissue, in accordance with the present disclosure;

FIG. 3A is a cross-sectional view of the surgical retractor of FIG. 1 partially inserted through tissue, in accordance with the present disclosure;

FIG. 3B is a cross-sectional view of the surgical retractor of FIG. 1 fully inserted through tissue, in accordance with the present disclosure;

FIG. 4 is a perspective view of a surgical retractor, where the ring is positioned substantially midway through the sleeve, in accordance with a second embodiment of the present disclosure;

FIG. 5 is a cross-sectional view of the surgical retractor of FIG. 4 illustrating the ring positioned substantially midway through the sleeve, in accordance with the second embodiment of the present disclosure;

FIG. 6 is a cross-sectional view of the ring of FIGS. 1-5, illustrating that the ring has at least two durometers, in accordance with the present disclosure.

FIG. 6A is an enlarged view of the cross-sectional view of FIG. 6, in accordance with the present disclosure;

FIG. 7 is a cross-sectional view of the ring of FIGS. 1-5, illustrating that the ring has at least two durometers, in accordance with the present disclosure.

FIG. 7A is an enlarged view of the cross-sectional view of FIG. 7, in accordance with the present disclosure;

FIG. 8 is a cross-sectional view of the ring of FIGS. 1-5, illustrating that the ring has at least two durometers, in accordance with the present disclosure.

FIG. 8A is an enlarged view of the cross-sectional view of FIG. 8, in accordance with the present disclosure;

FIG. 9 is a cross-sectional view of the ring of FIGS. 1-5, illustrating that the ring has at least two durometers, in accordance with the present disclosure.

FIG. 9A is an enlarged view of the cross-sectional view of FIG. 9, in accordance with the present disclosure;

FIG. 10 is a cross-sectional view of the ring of FIGS. 1-5, illustrating that the ring has at least two durometers, in accordance with the present disclosure.

FIG. 10A is an enlarged view of the cross-sectional view of FIG. 10, in accordance with the present disclosure;

FIG. 11 is a cross-sectional view of the ring of FIGS. 1-5, illustrating that the ring has at least two durometers, in accordance with the present disclosure.

FIG. 11A is an enlarged view of the cross-sectional view of FIG. 11, in accordance with the present disclosure;

Other features of the present disclosure will become apparent from the following detailed description, taken in conjunction with the accompanying drawings, which illustrate, by way of example, the principles of the present disclosure.

### DETAILED DESCRIPTION

Embodiments will now be described in detail with reference to the drawings wherein like numerals designate identical or corresponding elements in each of the several views. As is common in the art, the term "proximal" refers to that part or component closer to the user or operator, i.e., surgeon or physician, while the term "distal" refers to that part or component further away from the user. In the following description, well-known functions or constructions are not described in detail to avoid obscuring the present disclosure in unnecessary detail.

The example embodiments of the present disclosure illustrate a surgical retractor or surgical access port or wound protector, where at least one ring is of a variable durometer. The ring may be composed of at least two durometers in a plurality of shapes and sizes and configurations.

Reference will now be made in detail to embodiments of the present disclosure. While certain embodiments of the present disclosure will be described, it will be understood that it is not intended to limit the embodiments of the present disclosure to those described embodiments. To the contrary, reference to embodiments of the present disclosure is intended to cover alternatives, modifications, and equivalents as may be included within the spirit and scope of the embodiments of the present disclosure as defined by the appended claims.

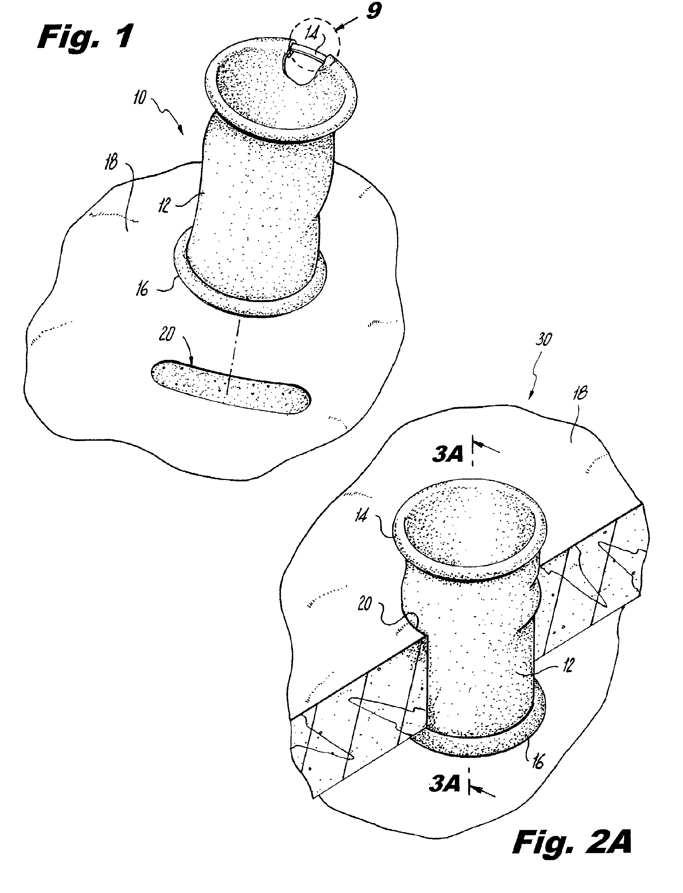

With reference to FIG. 1, a perspective view of a surgical retractor 10 illustrating at least one a ring, in accordance with the present disclosure is presented.

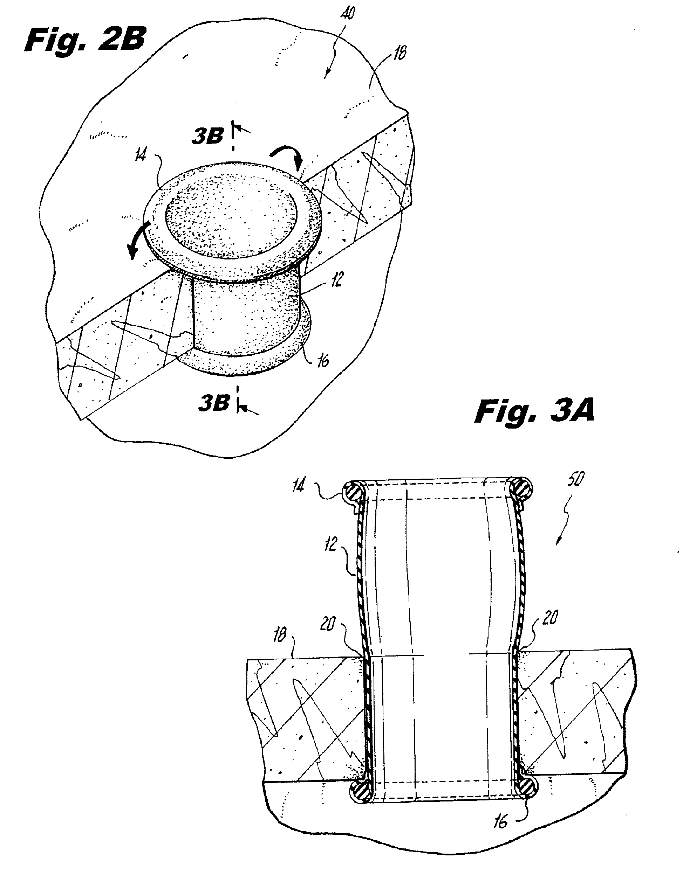

The surgical retractor 10 includes a first ring 14 and a second ring 16, which are fixed to a tubular member or sheath or liner or sleeve 12. The sleeve 12 has a generally cylindrical configuration with a proximal end and a distal end. The sleeve 12 may be flexible or collapsible. The second ring 16 is configured to be inserted within an incision 20 of tissue 18. The second ring 16 is stabilized or secured within the body of a patient (see FIGS. 2A, 2B), whereas the first ring 14 is stabilized or secured on the outer surface of the patient (e.g., around the vicinity of the incision 20 of tissue 18, see FIG. 2B).

The first and second rings 14, 16 may be O-rings that are made of a resilient or flexible material. First and second rings 14 and 16 may be preformed of elastomeric medical-grade polyurethane of sufficient hardness to retain the first and second rings 14, 16 expanded in place around the inner and outer rims or perimeters of the incision. The O-ring material is compliant enough to allow the first ring 14 to turn over 180° around its annular axis from the preformed configuration. The first and second rings 14, 16 may be color-coded with different colors, such as white and blue, for easier recognition of the correct O-ring to be inserted in the incision 20 of the tissue 18.

The sleeve 12 may be constructed from any pliable plastics film material. The first and second rings 14, 16 may be fixed at the distal ends of the sleeve. However, as shown in FIGS. 1-5, the sleeve 12 may slidably cooperate or move with respect to the first and second rings 14, 16. Either configuration is contemplated. Regardless of the configuration, the sleeve 12 allows for movement of the first ring 14 relative to the second ring 16. Additionally, the surgical retractor 10 is suitable for a range of incision sizes and is easily manufactured from any pliable plastics film material. It is also fairly easy to manipulate in use.

With reference to FIG. 2A, a perspective view 30 of the surgical retractor of FIG. 1 partially inserted through tissue, in accordance with the present disclosure is presented. With reference to FIG. 2B, a perspective view 40 of the surgical retractor of FIG. 1 fully inserted through tissue, in accordance with the present disclosure is presented.

The sleeve 12 has elastomeric properties, but in its natural, unstretched state, the first and second rings 14 and 16 are separated by a natural distance. By rolling ring 14 closer to the incision 20, the distance between the rings 14, 16 is reduced. FIG. 2A shows the sleeve 12 partially inserted through the incision 20 of tissue 18, whereas FIG. 2B shows the sleeve 12 fully inserted through the incision 20 of the tissue 18, such that the first ring 14 abuts the outer surface of the patient and the second ring 16 abuts the inner surface of the patient.

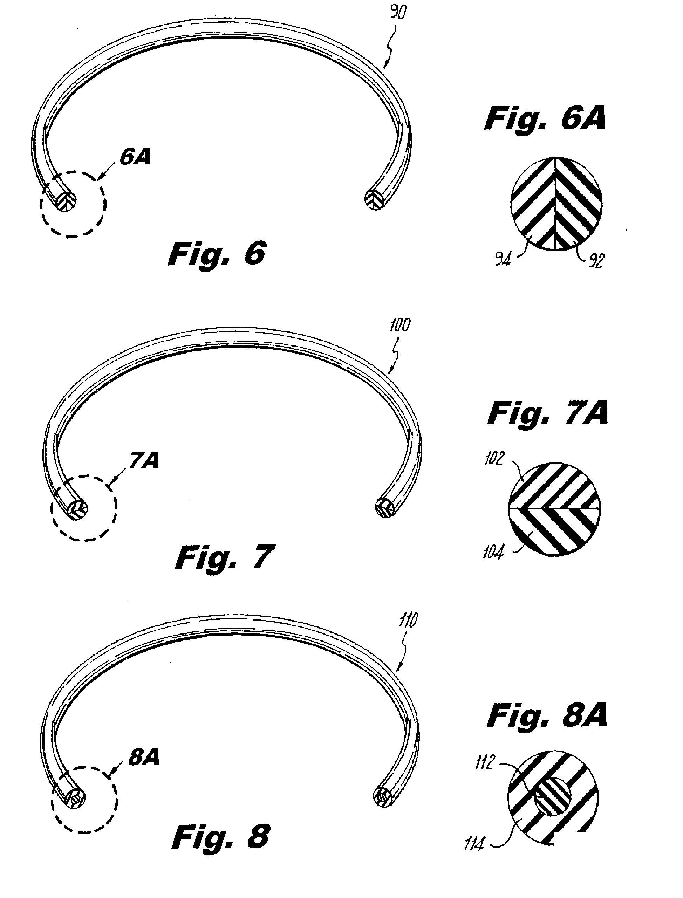

Since the diameters of the rings 14, 16 are greater than that desired for the incision 20 of tissue 18, they will have sufficient footing to maintain this tension between the two rings 14, 16. This tension is created by the elastic material that has been stretched and retained at a distance greater than the natural distance. It will be appreciated that in many embodiments, the sleeve 12 may be formed of a non-elastic sheathing material. In a similar manner, the rings 14 and 16 may be provided with a rigid configuration or alternatively may be formed of an elastomeric material or may be formed from at least two different durometers, as described below with reference to FIGS. 6-11.

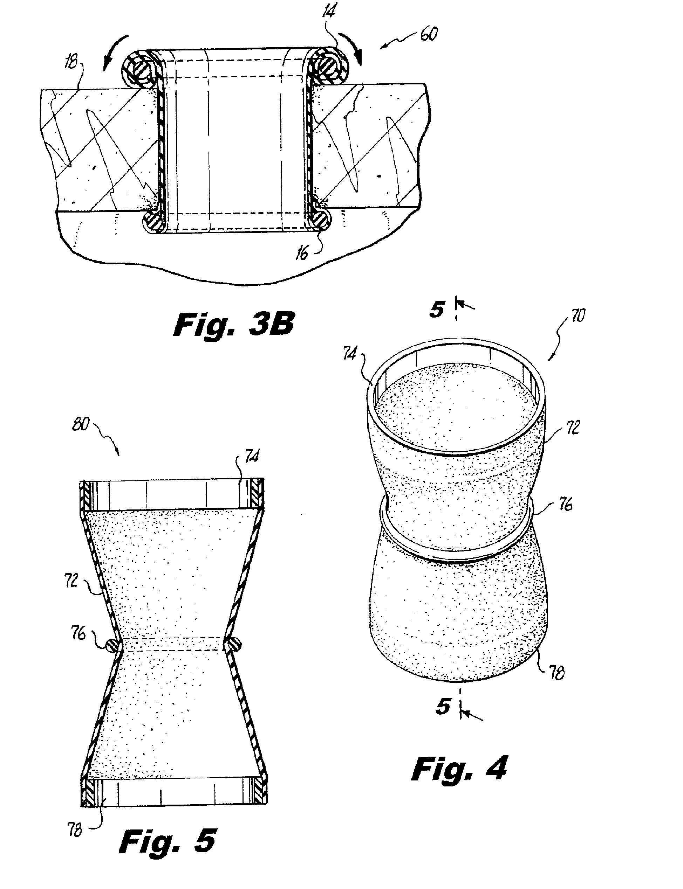

With reference to FIG. 3A, a cross-sectional view 50 of the surgical retractor of FIG. 1 partially inserted through tissue, in accordance with the present disclosure is presented. With reference to FIG. 3B, a cross-sectional view 60 of the surgical retractor of FIG. 1 fully inserted through tissue, in accordance with the present disclosure is presented.

As illustrated in FIGS. 3A and 3B, sleeve 12 is placed in the incision 20 of tissue 18, by squeezing the second ring 16 into a tight oblong shape and inserting it lengthwise through the incision 20 and letting it expand inside the peritoneum around the inner edge of the wound. Outer end portion of the sleeve 12 is gripped by the thumb and/or fingers at the first ring 14 and turned outwardly, in opposite directions, rolling sleeve 12 on the first ring 14 until it abuts the outer edge of the wound or incision 20 as shown in FIG. 3B. However, as a result of the elasticity of the liner material, any lengthwise adjustment not accommodated by the incremental adjustments cause the sleeve 12 in the wound between the first and second rings 14, 16 to be thereby drawn into contiguous contact with the edge margins of wound or incision 20 and hence with the wound walls to provide a self-retaining protective barrier during surgery, which is impervious to contaminating solids and fluids.

With reference to FIG. 4, a perspective view of a surgical retractor 70, where the ring is positioned substantially midway through the sleeve, in accordance with a second embodiment of the present disclosure is presented.

The surgical retractor 70 includes a sleeve 72 having a single ring 76. The ring 76 may be positioned substantially midway through the sleeve 72. The sleeve 72 may include a proximal end 74 and a distal end 78. The proximal end 74 and the distal end 78 may be sealing members.

With reference to FIG. 5, a cross-sectional view 80 of the surgical retractor 70 of FIG. 4 illustrating the ring positioned substantially midway through the sleeve, in accordance with the second embodiment of the present disclosure is presented.

FIG. 5 illustrates how the ring 76 engages the sleeve 72, while the ring 76 moves across the length of the sleeve 72. The ring 76 is constructed to be positioned on the top surface of an incision of tissue. It is envisioned that the ring 76 will be firmly stabilized or secured on the tissue.

With reference to FIGS. 6-11A, cross-sectional views of the ring of FIGS. 1-5, illustrating that the ring has two durometers, in accordance with the present disclosure are presented.

Ring 90 of FIGS. 6 and 6A includes a first durometer 92 and a second durometer 94. The second durometer may be greater than the first durometer or vice versa. The outer perimeter of the ring 90 includes the first durometer 92 and the inner perimeter includes the second durometer 94.

Ring 100 of FIGS. 7 and 7A includes a first durometer 102 and a second durometer 104. The second durometer may be greater than the first durometer or vice versa. The upper perimeter of the ring 100 includes the first durometer 102 and the lower perimeter includes the second durometer 104.

Ring 110 of FIGS. 8 and 8A includes a first durometer 112 and a second durometer 114. The second durometer may be greater than the first durometer or vice versa. The second durometer 114 is included within the first durometer 112. The second durometer 114 may be of a circular shape extending the entire distance of the ring 110. However, it is contemplated that the second durometer 114 extends only through one or more portions of the ring 110. In other words, the second material may define an inner tubular portion of the first ring 14 of FIG. 1 and the first material may define an outer tubular portion of the first ring 14 of FIG. 1 by encircling at least a portion of the second material.

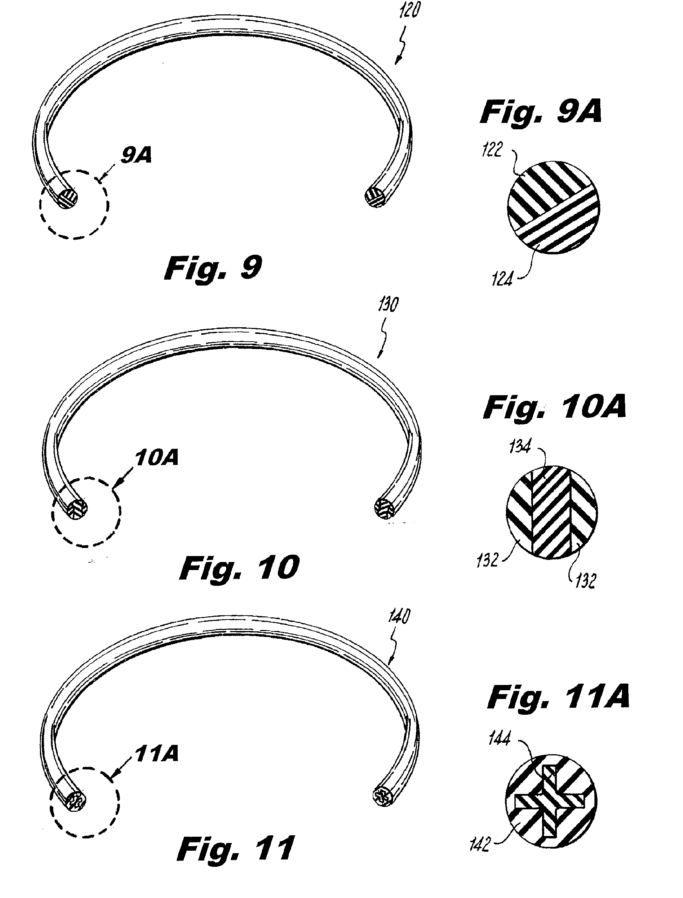

Ring 120 of FIGS. 9 and 9A includes a first durometer 122 and a second durometer 124. The second durometer may be greater than the first durometer or vice versa. The first and second durometers 122, 124 may be overlapping or interchangeably mixed throughout the length of the ring 120.

Ring 130 of FIGS. 10 and 10A includes a first durometer 132 and a second durometer 134. The second durometer may be greater than the first durometer or vice versa. The first durometer is positioned on the inner and outer edges or perimeters of the ring 130, whereas the second durometer 134 is sandwiched between the first durometers 132.

Ring 140 of FIGS. 11 and 11A includes a first durometer 142 and a second durometer 144. The second durometer may be greater than the first durometer or vice versa. The second durometer 144 is included within the first durometer 142. The second durometer 144 may be of a cross shape extending the entire distance of the ring 140. However, it is contemplated that the second durometer 144 extends through a portion of the ring 140. In other words, the second material may define an inner tubular portion of the first ring 14 of FIG. 1 and the first material may define an outer tubular portion of the first ring 14 of FIG. 1 by encircling at least a portion of the second material.

Therefore, in accordance with FIGS. 6-11A, the first ring 14 of FIG. 1 is formed by combining a first material having a first durometer with a second material having a second durometer. One skilled in the art may contemplate combining a plurality of different materials having a plurality of different durometers in a variety of shapes and sizes to provide for a tighter grip of the first ring 14 to the tissue 18.

It is also contemplated that the rings described herein include attached or detached portions of a first durometer and a second durometer across their lengths. In other words, a ring may include a first layer of a first durometer located next to a second layer of a second durometer, the layers being placed in alternating vertical fashion next to each other. Thus, the first ring may include multiple varying durometer portions, which are positioned adjacent to each other or separated from each other in alternating format.

The durometers of the rings described herein may be in a range of 40 to 100 Shore A. For example, the first material may have a hardness in a range of about 70-100 on a Shore A durometer, whereas the second material may have a hardness in a range of about 40-70 on a Shore A durometer or vice versa. Of course, one skilled in the art may contemplate combining materials having a vast array of durometers. The preferred material is urethane, but silicone could be used with some loss of stability after installation and adjustment. The size of the durometer affects both gripability for adjustment and stability after adjustment, since the larger the size of durometer for a given O-ring cross-sectional diameter, the less stability that exists.

In operation, the surgical retractor or wound protector of the example embodiments applies a force to the wound edges to achieve adequate exposure without causing ischemic injury to the wound edges. The surgical retractor or wound protector protects wound edges from cross infection or seeding by cancerous or otherwise malignant cells. Another advantage is that the surgical retractor or wound protector is disposed of after a single use, thereby obviating the need for cleaning and sterilization between uses. In addition, the surgical retractor or wound protector is simple to place into a desired position in a wound or natural bodily opening and easy to remove, especially without negating the benefits gained from use of the device as a wound protector. Finally, the surgical retractor or wound protector provides for a better, tighter grip adjacent the tissue since it is constructed from at least two different durometers in a variety of different shapes and sizes and configurations.

While the present disclosure has been explained by a detailed description of certain specific embodiments and has focused on medicine (human and veterinary) applications, there are many other industries, which may also benefit from the example embodiments of the present disclosure. It is understood that in other areas of industry, various modifications and substitutions may be made. These variations should be included within the scope of the appended claims, and also should include the equivalents of such embodiments.

It will be understood that there are to be no limitations as to the dimensions and shape of the surgical retractor or wound protector, including the body of the surgical instrument, or the materials from which the surgical instrument is manufactured. It is to be realized that the optimum dimensional relationships for the parts of the present disclosure, to include variations in size, materials, shape, form, function and manner of operation, assembly and use, are deemed readily apparent and obvious to one skilled in the art, and all equivalent relationships to those illustrated in the drawings and described in the specification are intended to be encompassed by the present disclosure.

While the present disclosure has been particularly shown and described with reference to the preferred embodiments, it will be understood by those skilled in the art that various modifications inform and detail may be made therein without departing from the scope and spirit of the present disclosure. Accordingly, modifications such as those suggested above, but not limited thereto, are to be considered within the scope of the present disclosure.

The invention may be described by reference to the following numbered paragraphs:-
1. A surgical retractor comprising:
   a tubular member having a proximal end and a distal end;
   a first ring secured at the proximal end of the tubular member; and
   a second ring secured at the distal end of the tubular member;
   wherein the first ring is formed by combining a first material having a first durometer and a second material having a second durometer.
2. The surgical retractor according to Paragraph 1, wherein the first ring and the second ring are O-rings.
3. The surgical retractor according to Paragraph 1, wherein at least one of the first ring and the second ring is flexible.
4. The surgical retractor according to Paragraph 1, wherein the tubular member is a sleeve.
5. The surgical retractor according to Paragraph 1, wherein the tubular member is flexible.
6. The surgical retractor according to Paragraph 1, wherein the second ring is formed by combining the first material having the first durometer and the second material having the second durometer.
7. The surgical retractor according to Paragraph 1, wherein the first material defines an outer periphery of the first ring and the second material defines an inner periphery of the first ring.
8. The surgical retractor according to Paragraph 7, wherein the first durometer is greater than the second durometer.
9. The surgical retractor according to Paragraph 7, wherein the first material has a hardness in a range of about 70-100 on a Shore A durometer, whereas the second material has a hardness in a range of about 40-70 on a Shore A durometer.
10. The surgical retractor according to Paragraph 1, wherein the first material has different elastomeric properties than the second material.
11. The surgical retractor according to Paragraph 1, wherein the second material defines an inner tubular portion of the first ring and the first material defines an outer tubular portion of the first ring by encircling at least a portion of the second material.
12. The surgical retractor according to Paragraph 1, wherein the first material combines with the second material so that a hollow inner portion is defined within the first ring.
13. The surgical retractor according to Paragraph 1, wherein the first ring includes multiple varying durometer portions.
14. A wound protector comprising:
   a distal ring;
   a proximal ring; and
   a flexible sleeve extending between the proximal ring and the distal ring;
   wherein the proximal ring is formed by combining a first material having a first durometer and a second material having a second durometer.
15. The wound protector according to Paragraph 14, wherein the distal ring and the proximal ring are O-rings.
16. The wound protector according to Paragraph 14, wherein at least one of the distal ring and the proximal ring is flexible.
17. The wound protector according to Paragraph 14, wherein the distal ring is formed by combining the first material having the first durometer and the second material having the second durometer.
18. The wound protector according to Paragraph 14, wherein the first material defines an outer periphery of the proximal ring and the second material defines an inner periphery of the proximal ring.
19. The wound protector according to Paragraph 18, wherein the first durometer is greater than the second durometer.
20. The wound protector according to Paragraph 18, wherein the first material has a hardness in a range of about 70-100 on a Shore A durometer, whereas the second material has a hardness in a range of about 40-70 on a Shore A durometer.
21. The wound protector according to Paragraph 14, wherein the first material has different elastomeric properties than the second material.
22. The wound protector according to Paragraph 14, wherein the second material defines an inner tubular portion of the proximal ring and the first material defines an outer portion of the proximal ring by encircling the second material.
23. The wound protector according to Paragraph 14, wherein the first material combines with the second material so that a hollow inner portion is defined within the proximal ring.
24. The wound protector according to Paragraph 14, wherein the proximal ring includes multiple varying durometer portions.
25. A flexible sealing ring for use with a surgical retractor to prevent cross-contamination during surgery of fluids and solids, the ring composed of at least two materials, the first material having a first durometer and the second material having a second durometer, where the first durometer is greater than the second durometer.
26. The flexible sealing ring according to Paragraph 25, wherein the ring is an O-ring.
27. The flexible sealing ring according to Paragraph 25, wherein the first material defines an outer periphery of the ring and the second material defines an inner periphery of the ring.
28. The flexible sealing ring according to Paragraph 25, wherein the second material defines an inner tubular portion of the ring and the first material defines an outer tubular portion of the ring by encircling the second material.
29. The flexible sealing ring according to Paragraph 25, wherein the first material combines with the second material so that a hollow inner portion is defined within the ring.
30. The flexible sealing ring according to Paragraph 25, wherein the ring includes multiple varying durometer portions.

## Claims

1. A surgical retractor comprising:
a tubular member having a proximal end and a distal end;
a first ring secured at the proximal end of the tubular member; and
a second ring secured at the distal end of the tubular member;
wherein the first ring is formed by combining a first material having a first durometer and a second material having a second durometer.

2. The surgical retractor according to Claim 1, wherein the first ring and the second ring are O-rings, preferably wherein at least one of the first ring and the second ring is flexible.

3. The surgical retractor according to Claim 1 or Claim 2, wherein the tubular member is a sleeve, preferably wherein the tubular member is flexible.

4. The surgical retractor according to any preceding Claim, wherein the second ring is formed by combining the first material having the first durometer and the second material having the second durometer.

5. The surgical retractor according to any preceding Claim, wherein the first material defines an outer periphery of the first ring and the second material defines an inner periphery of the first ring, preferably wherein the first durometer is greater than the second durometer,
preferably still wherein the first material has a hardness in a range of about 70-100 on a Shore A durometer, whereas the second material has a hardness in a range of about 40-70 on a Shore A durometer.

6. The surgical retractor according to any preceding Claim, wherein the first material has different elastomeric properties than the second material.

7. The surgical retractor according to any preceding Claim, wherein the second material defines an inner tubular portion of the first ring and the first material defines an outer tubular portion of the first ring by encircling at least a portion of the second material; and/or wherein the first material combines with the second material so that a hollow inner portion is defined within the first ring; and/or wherein the first ring includes multiple varying durometer portions.

8. A wound protector comprising:
a distal ring;
a proximal ring; and
a flexible sleeve extending between the proximal ring and the distal ring;
wherein the proximal ring is formed by combining a first material having a first durometer and a second material having a second durometer.

9. The wound protector according to Claim 8, wherein the distal ring and the proximal ring are O-rings; and/or wherein at least one of the distal ring and the proximal ring is flexible.

10. The wound protector according to Claim 8 or Claim 9, wherein the distal ring is formed by combining the first material having the first durometer and the second material having the second durometer; and/or wherein the first material defines an outer periphery of the proximal ring and the second material defines an inner periphery of the proximal ring.

11. The wound protector according to Claim 10, wherein the first durometer is greater than the second durometer, preferably wherein the first material has a hardness in a range of about 70-100 on a Shore A durometer, whereas the second material has a hardness in a range of about 40-70 on a Shore A durometer.

12. The wound protector according to any of Claims 8 to 11, wherein the first material has different elastomeric properties than the second material.

13. The wound protector according to any of Claims 8 to 12 wherein the second material defines an inner tubular portion of the proximal ring and the first material defines an outer portion of the proximal ring by encircling the second material; and/or wherein the first material combines with the second material so that a hollow inner portion is defined within the proximal ring; preferably wherein the proximal ring includes multiple varying durometer portions.

14. A flexible sealing ring for use with a surgical retractor to prevent cross-contamination during surgery of fluids and solids, the ring composed of at least two materials, the first material having a first durometer and the second material having a second durometer, where the first durometer is greater than the second durometer.

15. The flexible sealing ring according to Claim 14, wherein the ring is an O-ring; and/or wherein the first material defines an outer periphery of the ring and the second material defines an inner periphery of the ring; and/or wherein the second material defines an inner tubular portion of the ring and the first material defines an outer tubular portion of the ring by encircling the second material; and/or wherein the first material combines with the second material so that a hollow inner portion is defined within the ring, preferably wherein the ring includes multiple varying durometer portions.
